Europäisches Patentamt

European Patent Office

Office européen des brevets

(19)

(11) Veröffentlichungsnummer: **0 049 909**
**B1**

(12)     **EUROPÄISCHE PATENTSCHRIFT**

(45) Veröffentlichungstag der Patentschrift: **27.05.87**

(51) Int. Cl.⁴: **A 61 K 9/48,** A 61 J 3/07,
       A 61 J 5/00

(21) Anmeldenummer: **81108665.1**

(22) Anmeldetag: **28.10.78**

(80) Veröffentlichungsnummer der früheren
       Anmeldung nach Art. 76 EPÜ: **0 001 822**

(54) **Verbesserte pharmazeutische Präparate in Form von festen Dosiereinheiten sowie Verfahren zu ihrer Herstellung.**

(30) Priorität: **03.11.77 GB 4575577**

(43) Veröffentlichungstag der Anmeldung:
       **21.04.82 Patentblatt 82/16**

(45) Bekanntmachung des Hinweises auf die
       Patenterteilung:
       **27.05.87 Patentblatt 87/22**

(84) Benannte Vertragsstaaten:
       **BE CH DE FR GB NL SE**

(56) Entgegenhaltungen:
       **BE-A- 659 941**
       **DE-A-1 617 362**
       **DE-A-2 140 177**
       **FR-A-1 591 573**
       **FR-A-2 287 900**
       **GB-A- 709 283**
       **GB-A- 938 828**
       **GB-A- 976 510**
       **GB-A-1 264 511**
       **GB-A-1 414 967**
       **US-A-2 759 649**

(73) Patentinhaber: **HOECHST
       AKTIENGESELLSCHAFT
       Postfach 80 03 20
       D-6230 Frankfurt am Main 80 (DE)**

(72) Erfinder: **Walker, Stephen Ernest, Dr.
       Chetwode Close 3
       Buckingham (GB)**
       Erfinder: **Bedford, Keith
       Broad Street 35
       Newport Pagnell (GB)**
       Erfinder: **Eaves, Terence, Dr.
       Parkway 16
       Woburn Sands (GB)**

(56) Entgegenhaltungen:
       **US-A-2 949 941**
       **US-A-3 139 915**
       **US-A-3 870 089**

       **JOURNAL OF PHARMACEUTICAL SCIENCES,
       Band 64, Nr. 2, Februar 1975, Seiten 320-322,
       Amer. Pharm. Assoc., Washington, U.S.A., G.R.
       THOMPSON et al.: "Versatile unit for filling
       gelatin capsules with drugs or chemicals"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(56) Entgegenhaltungen:

CHEMICAL ABSTRACTS, Band 88, Nr. 26, Juni 1978, Seite 401, Nr. 197582n, Columbus, Ohio, U.S.A., A. CUINE et al.: "Filling capsules with viscous solutions of the active principles. I. Preliminary study of the excipients"

PHARMAZEUTISCHE INDUSTRIE, Band 40, Nr. 6, 1978, Ed. Cantor Verlag, Aulendorf, DE., A. CUINE et al.: "Das Einbringen viskoser Lösungen von Aktivestoffen in Hartgelatinekapseln"

CHEMICAL ABSTRACTS, Band 89, Nr. 14, 10. Februar 1978, Seite 529, Nr. 117690d, Columbus, Ohio, U.S.A., A. CUINE et al.: "Filling-capsules with viscous solutions of active principles. II. Rheological study of the fatty excipient"

CHEMICAL ABSTRACTS, Band 92, Nr. 24, Juni 1978, Seite 327, Nr. 203503p, Columbus, Ohio, U.S.A., A. CUINE et al.: "Filling geletin capsules with viscous solutions (or suspensions) of the active substance. III. Active substance formulation"

**Beschreibung**

Die Erfindung betrifft die Herstellung pharmazeutischer Präparate in Hartkapselform, vorzugsweise in Hartgelatinekapselform.

Es ist wichtig, daß pharmazeutische Präparate eine bekannte Wirkstoffmenge enthalten, und in vielen Fällen ist eine genaue Dosierung erforderlich, insbesondere bei hochwirksamen, in geringen Mengen zu verabreichenden Arzneimittel. Zur peroralen Verabreichung werden feste Dosiereinheitsformen im allgemeinen flüssigen Formen vorgezogen, da sie eine Verabreichung in genauer Dosis gestatten, im allgemeinen leichter zu verabreichen sowie Häufig stabiler sind. Hartgelatinekapseln sind eine verhältnismäßig teure, aber höchst annehmbare feste Dosiereinheitsform, wobei die Gelatinehüllen im allgemeinen mit Feststoff gefüllt werden. Die Verabreichung von Fest stoffen zu Granulaten und Pulvern zur Kapselfüllung stellt jedoch gewisse technische Probleme; beispielsweise ist es schwierig, eine einheitliche Dispersion des Wirkstoffs in den verwendeten Trägern bereitszustellen.

Vor kurzem wurde eine Kapselfüllmaschine beschrieben (DOS 26 12 472), womit pastenförmige oder halbfeste Stoffe in Hartgelatinekapselhüllen dosiert werden können. Der pastenförmige oder halbfeste Stoff wird als Schicht einheitlicher Dicke und Dichte extrudiert, und ein Rohrglied wird senkrecht durch die gesamte Dicke der Schicht eingeführt. Wegen der pastenartigen Konsistenz des zu dosierenden Stoffes verbleibt ein Pfropfen davon mit dem Glied und wird dann daraus in einer Kapselhülle ausgepreßt und mittels Druckluft positioniert. Dieses System besitzt mehrere Nachteile: die Genauigkeit der Dosierung hängt davon ab, daß die erhaltene, extrudierte Stoffschicht eine sehr einheitliche Dicke besitzt, und die Probleme der Vermischung des Arzneimittels mit der pastenartigen Grundmasse müssen gelöst werden; das Rohrglied muß mit einem Verhältnismäßig komplizierten Mechanismus gesteuert werden, der das Glied aus seiner Stellung über der extrudierten Schicht in seine Stellung über der Kapselhülle schwenkt; das Ausstoßen des Stoffpfropfens aus dem Glied ist schwierig; und bei Verwendung eines öligen Stoffes kann dieser gegebenenfalls an der Fuge der Kapsel ausfließen.

Die Verwendung eines flüssigen Stoffes in Kapseln bringt Probleme, da die Flüssigkeit gegebenenfalls zwischen den Hälften der Kapselhülle ausfließt. Dies ist vermeidbar, wenn man ein Band um die Fuge legt: dies führt jedoch zu einer zusätzlichen Stufe im Füllverfahren und erhöht daher die Herstellungskosten. In Weichgelatinekapseln kann man allerdings Flüssigkeiten verwenden. Weichgelatinekapseln vereinigen den Vorteil einer festen Dosiereinheitsform mit dem Vorteilen einer Flüssigkeit im Hinblick darauf, daß eine einheitlichen Vermischung des Wirkstoffs im Träger leicht zu erzielen ist und das so erhaltene Gemisch mit großer Genauigkeit dosiert werden kann. Sie besitzen jedoch den Nachteil, daß hochspezialisierte Vorrichtungen zu ihrer Herstellung erforderlich sind, welche daher im allgemeinen eher von Unterlieferanten als von der pharmazeutischen Firma selbst durchgeführt wird. Dies führt zu höheren Kosten als bei Hartgelatinekapseln und hat ferner weitere Nachteile, beispielsweise Probleme bezüglich Forschung und Entwicklung neuer Pharmazeutika.

Es sollte nicht nur die Wirkstoffmenge in einem pharmazeutischen Präparat bekannt sein, sondern auch der Grad und die Geschwindigkeit seiner Abgabe in vivo, das heißt seine biologische Verfügbarkeit. Über die Formulierung von Präparaten mit hoher biologischer Verfügbarkeit des Wirkstoffs sowie in vielen Fällen spezifischen Abgabeeigenschaften bestehen Arbeiten. Seit der 1960er Jahren wurden laufend Arbeiten über die Anwendung fester Lösungen, fester Dispersionen und aus dem Wirkstoff in einem geeigneten Träger, z.B. Harnstoff oder einem Polyäthylenglykol, bestehender eutektischer Gemische durchgeführt. Es hat sich gezeigt (siehe beispielsweise Journal of Pharmaceutical Sciences 60, 1281 (1971)), daß solche Systeme unter Verwendung von hydrophilen Trägern, z.B. Polyäthyleneglykolen, zu einer herkömmlichen festen Formulierungen weit überlegenen biologischen Verfügbarkeit führen können, während man zur Verzögerung der Abgabe des wirksamen Prinzips hydrophobe Träger verwenden kann. Als hydrophobe Grundmassen eignen sich unter anderem beispielsweise niedrigschmelzende Wachse, Öle sowie wasserunlösliche Polymere. Im letzteren Fall kann das wasserunlösliche Polymer im Gemisch mit einem flüssigen hydrophilen Träger vorliegen: es wurden Weichgelatinekapseln mit verzögerter Abgabe beschrieben, in welchen der flüssige Kern Polyäthylenglykol 600 und einige Prozent Polyvinylacetat enthält; mit der Auflösung des Polyäthylenglykols in den Magensäften führt das vorhandene Wasser zur Fällung des Polyvinylacetats als Feststoff; wodurch die Abgabe des Wirkstoffs verzögert wird. Mit zunehmenden Anteil an vorhandenem Polyvinylacetat nimmt die Abgabegeschwindigkeit des Wirkstoffs ab.

Der Nachteil bei der großtechnischen Verwendung fester Dispersion oder eutektischer Gemische besteht jedoch darin, daß sie allgemein sehr schlechte Verarbeitungseigenschaften besitzen, indem sie im allgemeinen klebrige glasartige Massen mit schlechtem Fließverhalten darstellen, die mit der herkömmlichen Tablettier- oder Kapselfülltechnik seht schwer zu verarbeiten sind.

Gegenstand vorliegender Erfindung ist in Verfahren zur Herstellung pharmazeutischer Präparate in Dosiereinheitsform, bei dem Hartgelatinekapseln mit flüssigen, nach ihrem Einbringen erstarrenden Heilmittelwirkstoff enthaltenden Trägersubstanzen gefüllt werden, welches dadurch gekennzeichnet ist, daß der flüssige Träger ein thixotropes Gemisch ist, das innerhalb der Kapsel soweit erstarrt oder soweit geliert, daß die eingefüllte Mischung ihr flüssiges Fließverhalten verliert.

Der den Wirkstoff enthaltende flüssige Träger ist also ein thixotropes Gemisch, das sich während des Abfüllvorgangs wie eine Flüssigkeit, aber innerhalb der Kapselhülle wie ein Feststoff verhält.

Durch geeignete Auswahl des Trägermaterials kann man die Auflösung des Arzneimittels in vivo fördern und die biologische Verfügbarkeit des Arzneimittels erhöhen. In einer Lösung oder Dispersion liegt das Arzneimittel in einer sehr viel feinverteilteren Form vor, als durch einfaches Vermischen des Arzneimittels mit einem festen Träger erhältlich ist. Ist der Träger in Magansäften sehr leicht lösliche, so löst er sich bei der Verarbreichung sehr schnell auf, und das Arzneimittel, das in sehr feinverteilter Form vorliegt, wird leicht und schnell resorbiert. Bei Arzneimitteln, die in Magensäften nicht sehr leicht löslich sind, stellt die einen großen Vorteil dar.

Ist der Träger dagegen in Magensäften nicht leicht löslich, so erhält man ein System mit verzögerter Abgabe; zu derartigen Trägern gehören mit Thioxotropiermitteln gelierte Öle, zum Beispiel Paraffinöl.

Geeignete Geliermittel, die zur Herstellung thixotroper Mischungen zur Verfügung stehen, sind unter anderem hydriertes Rizinusöl (z.B. [R]Thixcin) und kolloidales Siliciumdioxyd (z.B. [R]Aerosil).

Da das erfindungsgemäße Verfahren eine flüssigen Träger verwendet, entfallen die Probleme einer einheitlichen Vermischung eines festen Arzneimittels mit einem festen Träger. Durch einfaches Rühren des flüssigen Gels erhält man ein homogenes Gemisch bzw. eine homogene Lösung. Dies ist von besonderer Bedeutung bei Systemen, wo wirksames Vermischen von Feststoffen schwierig ist: zum Beispiel besitzten gewisse niedrig-dosierte perorale Empfängnisverhütungsmittel eine Teilchengröße und Gestalt, welche die Herstellung fester Dosiereinheitsformen mit befriedigend einheitlichem Gehalt sehr erschweren. Es wurde zum Beispiel berichtet, daß der Steroidgehalt in niedrig-dosierten Empfängnisverhütungstabletten in ziemlich weiten Grenzen schwankt (vgl. von P.C. Fait und G.H. King am Techicon Colloqium über "Automated Analysis in the Pharmaceutical Industry [Automatisierte Analyse in der Pharmazeutischen Industrie]", Bloomsbury Centre Hotel, London, 23. April 1970, gehaltener Vortrag). Solche Steroide füllt man daher sehr zweckmäßig nach dem erfindungsgemäßen Verfahren in Kapselhüllen ab. Das erfindungsgemäße Verfahren ist beispielsweise ebenfalls zur Herstellung von Digoxin enthaltenden Kapseln verwendbar; die bei dessen Auflösung auftretenden Probleme haben in jüngster Zeit erhebliches Interesse erweckt.

Ein großer Vorteil des erfindungsgemäßen Verfahrens besteht darin, daß man zur Füllung der Kapseln herkömmliche Hartgelatinekapselfüllmaschinen mit nur kleinem Umbau verwenden kann. Solche Maschinen zur Abfüllung von Pulvern in einer Kapselhülle besitzen einen Abfüllkopf, der einen Pulverpropfen aufhebt, und diesen in eine Kapselhülle fallen läßt, die sich auf einem Drehtisch befindet, welcher dann rotiert, um eine neue Hülle in die Abfüllstellung zu bringen. Durch Ersatz des Abfüllkopfes durch eine Vorrichtung zum Dosieren einer Flüssigkeit anstelle eine Pulvers lassen sich die Maschinen leicht zur Durchführung des erfindungsgemäßen Verfahrens umbauen.

Die Kapselfüllmaschine, welche vorzugsweise zum Eindosieren der Flüssigkeit verwendet wird, ist mit einem Drehtisch, mit einem Detektorsystem und einem zur Dosierung von Flüssigkeiten geeigneten Abfüllkopf ausgerüstet Als Abfüllköpf lassen sich beispielsweise eine nachfüllbare Spritze, ein Schlauchpumpe oder ein Präzisionsspritzgerät verwenden. Dabei hat sich ein mit Druckluft betätigtes Präzisionsspritzgerät besonders bewährt, da es eine exakte und wiederholbare Dosis Flüssigkeit liefert.

In einer herkömmlichen Hartgelatinekapselfüllmaschine ist üblicherweise unter der Abfüllstation ein Aufnahmegefäß vorgesehen, damit das ausgegebene Material gesammelt wird, falls die Maschine keinen Kapselkörper unter den Abfüllkopf stellt. Wird jedoch die Maschine zur Ausgabe einer Flüssigkeit umgebaut, so ist dieses Sammelsystem unzureichend, da die Flüssigkeit den Apparat verschmutzt. Dies läßt sich durch Einbau eines Detektorsystem vermeiden, welches beispielsweise auf einer Photozelle beruht, so daß der Abfüllmechanismus nur dann Flüssigkeit ausgibt, wenn sich eine Kapselhülle in der richtigen Stellung unter dem Abfüllmechanismus befindet. Vorzugsweise wird dieses System erweitert, um die Gegenwart beschädigter Hüllen festzustellen und deren Abfüllung zu verhindern. Das Detektorsystem kann beispielsweise einen Mikroschalter betätigen, welcher den Drehtisch und den Abfüllmechanismus steuert; wenn keine Kapselhülle unter dem Abfüllmechanismus steht, wird vorzugsweise keine Flüssigkeit ausgegeben, und der Drehtisch rotiert, um die nächste Kapselhülle hinzustellen, so daß der Abfüllgang nicht ernsthaft unterbrochen wird.

Die Kapselfüllmaschine kann sowohl für intermittierende Bewegung als auch für kontinuierliche Bewegung ausgebildet sein.

Kapseln, die miteinander unverträgliche Arzneimittel enthalten, kann man dadurch herstellen, daß man eine Flüssigkeit mit einem Arzneimittel in die Hülle dosiert und nach Erstarrung eine Flüssigkeit mit einem zweiten Arzneimittel in die Hülle dosiert. Ebenfalls können mit einem Gel dosierte Hüllen auch Kügelchen, Granulate, Tabletten oder ein Pulver enthalten.

Somit bietet das erfindungsgemäße Verfahren die Vorteile einer Flüssigkeit im Hinblick auf einheitlichen Gehalt des entstandenen Produkts sowie die Vorteile eines Feststoffs im Hinblick auf bequeme Verabreichung und Stabilität des Präparates. Ferner lassen sich auch die Vorteile der Verwendung fester Lösungen, fester Dispersionen und eutektischer Gemische im Hinblick auf gesteuerte Abgabe des Arzneimittels und dessen biologische Verfügbarkeit ausnutzen. Ein weiterer Vorteil besteht darin, daß eine für das erfindungsgemäße Verfahren verwendete Formulierung im allgemeinen sehr viel einfacher ist als eine bei der Verwendung herkömmlicher Tablettier- oder Kapselfülltechnik erforderliche Formulierung, wobei im allgemeinen die Gegenwart von verschiedenen als Fließhilfsmittel wirkenden Trägern, Schmiermitteln und Streckmitteln zur Verbesserung des Fließverhaltens der Formulierung notwendig ist. Bei dem erfindungsgemäßen Verfahren ist die flüssige Formulierung natürlich selbstschmierend und kann sehr einfach aufgebaut, sein beispielsweise aus einem Öl, einem Thixotropiermittel und dem Arzneimittel.

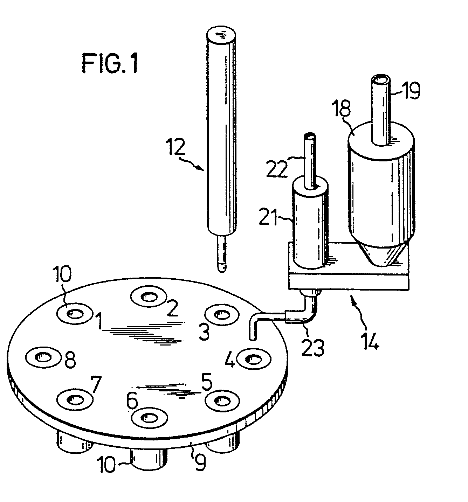

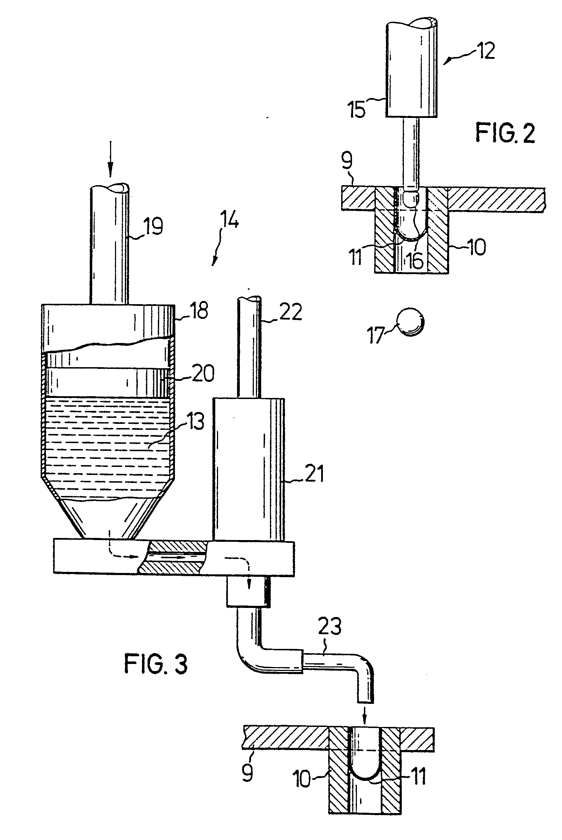

Eine Kapselfüllmaschine, welche bei dem erfindungsgemäße Verfahren verwendet werden kann, sei nun anhand der beigefügten Zeichnungen beispielshaft beschrieben. Es zeigen: Figur 1 den Drehtisch einer Kapselfüllmaschine und deren dazugehörige Abfüllvorrichtung; Figur 2 das Kapseldetektor system der Maschine in Figure 1; und Figur 3 das in der Maschine in Figur 1 verwendete Präzisionsspritzgerät.

Figur 1 zeigt den Drehtisch 9 der Kapselfüllmaschine mit acht um deren Umfang angeordneten Stationen. Diese Stationen umfassen die Kapseleinführstation 1, Trennstation 2, Detektorstation 3, Abfüllstation 4, Kapselschließstation 5, Auswerferstation 6, Regelstation 7 sowie eine Station 8, die beim erfindungsgemäßen Verfahren nicht benutzt wird.

Bei der Kapseleinführstation 1 wird eine leere Kapselhülle in einen Kapselhalter (10) auf dem Drehtisch 9 gestellt. Bei der Trennstation 2 wird der Kapseldeckel von Kapselkörper 11 getrennt. Über der Detektorstation 3 ist ein Detektorsystem 12 angeordnet. Bei der Abfüllstation 4 wird flüssiges Füllmaterial 13 mittels eine Präzisionsspritzgerätes 14 in den Kapselkörper 11 eingespritzt. Bei Station 5 wird die Kapselhülle durch Wiederaufsetzen des Deckels auf den Kapselkörper 11 verschlossen, und die so erhaltene Kapsel wird bei der Auswerferstation 6 aus dem Drehtisch ausgeworfen. Das durch den Drehtisch 9 aktivierte Betätigungsglied (ein Mikroschalter, nicht dargestellt) für das Präzisionsspritzgerät 14 befindet sich an der Regelstation 7. Das Detektorsystem 12 ist in Figur 2 mehr im einzelen dargestellt. Es besteht aus einer Stützstange (15), die auf nicht dargestellte Weise in einer senkrechten Ebene beweglich ist, einer lichtemitierenden Diode 16 und einem Detektor 17. Ist im Kapselhalter 10 kein Kapselkörper 11 vorhanden, so erreicht Licht aus der lichtemitierenden Diode 16 den Detektor 17 und betätigt dadurch den Mikroschalter (nicht dargestellt) an der Steuerstation 7, was den Abfüllgung verzögert und somit ein Verschütten des flüssigen Füllmaterials 13 verhindert.

Das Präzisionsspritzgerät 14 ist in Figur 3 dargestellt. Es besteht aus einem Behälter 18 für flüssiges Füllmaterial 15, mit einem Einlaß für Druckluft, die einen Kolben 20 betätigt; ein Ventilwerk 21, in dem das Ventil mittels durch einen Einlab 22 zugeführte Druckluft geöffnet oder geschlossen wird, ermögliche, daß das flüssige Füllmaterial 13 in Richtung der Pfeile in eine Abfülldüse 23 und dann in den Kapselkörper 11 fließt.

Der Behälter 18 ist mit einer Heizvorrichtung (nicht dargestellt) versehen, beispielswese einem Heizband oder einem Heißluftgebläse. Die abgegebene Menge flüssigen Füllmaterials 13 hängt vom am Einlaß 19 angewandten Luftdruck und einem Zeitgeber (nicht dargestellt) ab, der von dem Mikroschalter (nicht dargestellt) an der Regelstation 7 gesteuert wird. Ferner ist sie auch von Größen wie der Viskosität des flüssigen Füllmaterial 13 abhängig.

Zur Durchführung des erfindungsgemäßen Verfahrens kann das Präzisionsspritzgerät auch abgewandelt werden, zum Beispiel kann der Kolben 20 fehlen.

Das nachfolgende Beispiel erläutet die Erfindung.

### Beispiel

| Formel | mg/Kapsel |
|---|---|
| Kolloidales Siliciumdioxyd (z.B. (R)AEROSIL 200) | 12,4 |
| Hydriertes Rizinusöl (z.B. (R)THIXCIN R) | 18,6 |
| Paraffinöl | 589,0 |
| GESAMTFÜLLGEWICHT | 620 mg |

Das Thixcin und Aerosil werden unter Verwendung eines Schnellmischers im Paraffinöl dispergiert, und zur Entwicklung des Gels wird das Gemisch auf 40°C erwärmt. Das thixotrope Gel wird unter Verwendung eines Präzisionsspritzgerätes in Kapselhüllen abgefüllt. Für ein Füllgewicht von 620 mg läßt sich eine relative Standard-Abweichung von 1,4 % erreichen.

**Patentansprüche**

1. Verfahren zur Herstellung von pharmazeutischen Präparaten in Dosiereinheitsförm, bei dem Hartgelatinekapseln mit flüssigen, nach ihrem Einbringen erstarrenden, Heilmittelwirkstoff enthaltenden Trägersubstanzen gefüllt werden, dadurch gekennzeichnet, daß der flüssige Träger ein thixotropes Gemisch ist, das innerhalb der Kapsel soweit erstartt oder soweit geliert, daß die eingefüllte Mischung ihr flüssiges Fließverhalten verliert.

2. Verfahren gemäß Anspruch 1, dadurch gekennzeichnet, daß das Gemisch ein thixotropes Geliermittel enthält.

3. Verfahren nach einem der Ansprüche 1 oder 2, dadurch gekennzeichnet, daß als thixotropes Geliermittel hydriertes Rizinusöl oder kolloidales Siliciumdioxyd vorliegt.

4. Verfahren nach einem der Ansprüche 1 bis 3, dadurch gekennzeichnet, daß der den Wirkstoff enthaltende flüssige Träger unter Verwendung einer Kapselfüllmaschine, die mit eine, Drehtisch, mit einem Detektorsystem und einem zur Dosierung einer Flüssigkeit geeigneten Abfüllkopf ausgerüstet ist, in die Hartgelatinekapsel eindosiert wird.

5. Verfahren nach einem der Anspruch 1 bis 4, dadurch gekennzeichnet, daß der den Wirkstoff enthaltende flüssige Träger unter Verwendung einer Kapselfüllmaschine, deren Abfüllkopf eine nachfüllbare Spritze, ein Schlauchpumpe oder in Präzisionsspritzgerät ist, in die Hartgelatinekapsel eindosiert wird.

6. Verfahren nach Anspruch 5, dadurch gekennzeichnet, daß die Dosierung in einer Hartgelatinekapselschnellfüllmaschine erfolgt.

7. Pharmazeutische Zusammensetzung in Dosiereinheitsform bestehend aus einer Hartgelatinekapsel, die Wirkstoff homogen verteilt in einem, in flüssiger Form eingefüllten erstarrten Träger enthält, dadurch gekennzeichnet, daß der Träger ein thixotropes Gemisch ist, das soweit et starrt oder geliert ist, daß es sein flüssiges Fließverhalten verloren hat.

8. Pharmazeutische Zusammensetzung in Dosiereinheitsform bestehend aus einer Hartgelatinekapsel, die Wirkstoff homogen verteilt in einem in flüssiger Form eingefüllten, erstarrten Träger enthält, dadurch gekennzeichnet, daß sie als Träger ein ein thixotropes Geliermittel enthaltendes Gemisch enthält, das so weit erstarrt oder geliert ist, daß es sein flüssiges Fließverhalten verloren hat.

**Revendications**

1. Procédé pour la préparation de compositions pharmaceutiques sous forme d'unités de dosage, dans lequel des gélules de gélatine dure sont remplies avec des substances liquides formant véhicules contenant un médicament actif et se solidifiant après leur incorporation, caractérisé en ce que le véhicule liquide est une mélange thixotropique qui se solidifie ou se gélifie à l'intérieur de la gélule, de sorte que le mélange introduit perd son comportement liquide.

2. Procédé selon la revendication 1, caractérisé en ce que le mélange contient un agent gélifiant thixotrope.

3. Procédé selon l'une des revendications 1 ou 2, caractérisé en ce que, comme agent gélifiant thixotrope, on utilise l'huile de ricin hydrogénée ou la silice colloïdale.

4. Procédé selon l'une des revendications 1 à 3, caractérisé en ce que le véhicule liquide contenant la substance active est dosé dans la gélule de gélatine dure, en utilisant une machine à remplir les gélules, équipée d'une table rotative, d'un système détecteur et d'une tête de remplissage permettant le dosage d'un liquide.

5. Procédé selon l'une des revendications 1 à 4, caractérisé en ce que le véhicule liquide contenant la substance active est dosé dans le gélule de gélatine dure, en utilisant une machine à remplir les gélules dont la tête de remplissage est une seringue rechargeable, un pompe tubulaire ou un injecteur de précision.

6. Procédé selon la revendication 5, caractérisé en ce que le dosage a lieu dans une machine à remplissage rapide des gélules de gélatine dure.

7. Composition pharmaceutique sous forme d'unité de dosage, constituée d'une gélule de gélatine dure qui contient la substance active répartie de manière homogène dans un véhicule solidifié, mais introduit sous forme liquide, caractérisée en ce que le véhicule est un mélange thixotropique qui est solidifié ou gélifié de telle sorte qu'il a perdu son comportement liquide.

8. Composition pharmaceutique sous forme d'unité de dosage, constituée d'une gélule de gélatine dure qui contient la substance active répartie de manière homogène dans un véhicule solidifié, mais introduit sous forme liquide, caractérisée en ce que, comme véhicule, elle contient un mélange comportant un agent gélifiant thixotropique, qui est solidifié ou gélifié de telle sorte qu'il a perdu son comportement liquide.

**Claims**

1. Process for the manufacture of pharmaceutical preparations in unit dosage form, in which hard gelatin capsules are filled with liquid carrier substances that solidify after being introduced and contain a pharmaceutically active substance, characterised in that the liquid carrier is a thixotropic mixture which solidifies or gels inside the capsule to such an extent that the mixture introduced loses its liquid flow behavior.

2. Process according to claim 1 characterized in that the mixture contains a thixotropic gelling agent.

3. Process according to any one of claims 1 or 2 characterized in that hydrogenated castor oil or colloidal silicon dioxide is present as the thixotropic gelling agent.

4. Process according to any one of claims 1 to 3, characterised in that the liquid carrier containing the active substance is dosed into the hard gelatin capsule using a capsule-filling machine equipped with a turntable, a detection system and a filling head suitable for dosing a liquid.

5. Process according to any one of claims 1 to 4, characterised in that the liquid carrier containing the active substance is dosed into the hard gelatin capsule using a capsule-filling machine, the filling head of which is a refillable syringe, a tube pump or a precision shot dispenser.

6. Process according to claim 5, characterised in that the dosing operation takes place in a highspeed hard gelatin capsule-filling machine.

7. Pharmaceutical composition in unit dosage form, comprising a hard gelatin capsule that contains the active substance homogeneously dispersed in a solidified carrier introduced in liquid form, characterised in that the carrier is a thixotropic mixture that has solidified or gelled to such an extent that it has lost its liquid flow behavior.

8. Pharmaceutical composition in unit dosage form comprising a hard gelatin capsule that contains the active substance homogeneously dispersed in a solidified carrier introduced in liquid form, characterized in that it contains as carrier a mixture that contains a thixotropic gelling agent that has solidified or gelled to such an extent that it has lost its liquid flow behavior.

FIG.1

0 049 909

FIG. 2

FIG. 3